# EUROPEAN PATENT APPLICATION

(11) **EP 4 119 096 A2**
(43) Date of publication of application: **18.01.2023**
(21) Application number: 22184379.0
(22) Date of filing: 12.07.2022
(51) Int. Cl.: A61F 2/24, A61F 2/95

(54) **HYDRAULIC TENSION AND COMPRESSION IMPLANT DEPLOYMENT AND RECAPTURE SYSTEM**

(30) Priority: 16.07.2021 US 202163222855 P; 23.06.2022 US 202217808436
(71) Applicant: Medtronic, Inc., Minneapolis, MN 55432 (US)
(72) Inventor: Griswold, Erik C., Santa Rosa (US); Bruner, Kenny D., Santa Rosa (US); Dwork, Joshua, Santa Rosa (US); Haynes, Austin, Santa Rosa (US)
(74) Representative: Zimmermann & Partner Patentanwälte mbB

(57) **Abstract**

An apparatus for hydraulic deployment and recapture of an implant includes a hydraulic implant uncovering mechanism and a hydraulic implant re-covering mechanism. The implant uncovering mechanism and the implant re-covering mechanism are independently actuateable with a fluid, and the implant uncovering mechanism is coupled to the implant re-covering mechanism by a flexible elongate tether.

## Description

This application claims the benefit of U.S. Provisional Application No. 63/222,855, filed July 16, 2021, the disclosure of which is incorporated herein by reference in its entirety.

### BACKGROUND

Mitral heart valves can be affected by conditions such as, for example, mitral valve regurgitation, mitral valve prolapse and mitral valve stenosis.

Mitral valve regurgitation is abnormal leaking of blood from the left ventricle into the left atrium caused by a disorder of the heart in which the leaflets of the mitral valve fail to coapt into apposition at peak contraction pressures. The mitral valve leaflets may not coapt sufficiently because heart diseases often cause dilation of the heart muscle, which in turn enlarges the native mitral valve annulus to the extent that the leaflets do not coapt during systole. Abnormal backflow can also occur when the papillary muscles are functionally compromised due to ischemia or other conditions. More specifically, as the left ventricle contracts during systole, the affected papillary muscles do not contract sufficiently to effect proper closure of the leaflets.

Mitral valve prolapse is a condition when the mitral leaflets bulge abnormally up into the left atrium. This can cause irregular behavior of the mitral valve and lead to mitral valve regurgitation. The leaflets may prolapse and fail to coapt because the tendons connecting the papillary muscles to the inferior side of the mitral valve leaflets (chordae tendineae) may tear or stretch. Mitral valve stenosis is a narrowing of the mitral valve orifice that impedes filling of the left ventricle in diastole.

In some cases, the entire mitral valve can be replaced by implanting mechanical valves or biological tissue into the heart in place of the native mitral valve. This replacement procedure can require large open thoracotomies and are thus very painful, have significant morbidity, and require long recovery periods. In addition, the durability of the devices or improper sizing of annuloplasty rings or replacement valves may cause additional problems for the patient.

Less invasive procedures have sought to replace the mitral valve by percutaneously delivering a prosthetic heart valve device into a mitral valve of a patient. However, percutaneous mitral valve replacement can be difficult because the mitral valve annulus has a non-circular D-shape or kidney-like shape, with a non-planar, saddle-like geometry often lacking symmetry. The complex and highly variable anatomy of mitral valves makes it difficult to design a mitral valve prosthesis that conforms well to the native mitral annulus of specific patients. As a result, the prosthesis may not fit well with the native leaflets and/or annulus, which can leave gaps that allow backflow of blood to occur. For example, placement of a cylindrical valve prosthesis in a native mitral valve may leave gaps in commissural regions of the native valve through which perivalvular leaks may occur.

Percutaneous hydraulic delivery systems may be used for mitral valve replacement procedures. Some hydraulic systems may use a catheter delivered intravascularly into the heart, or through a cannula inserted through the heart wall. Generally, delivery systems facilitate controlled delivery of a prosthetic heart valve device using trans-apical or trans-septal delivery approaches, and may allow re-sheathing of the prosthetic heart valve device within a delivery capsule after partial deployment of the prosthetic device to reposition and/or remove the prosthetic device within the mitral valve annulus.

Hydraulic control and actuation for both proximal and distal movement of a capsule housing overlying a prosthetic heart valve device may provide for controlled delivery of the prosthetic heart valve device and inhibit uncontrolled movement of the delivery system resulting from forces associated with expansion of the prosthetic heart valve device (e.g., axial jumping, self-ejection, etc.). In addition, the hydraulic delivery systems may inhibit longitudinal translation of the prosthetic heart valve device relative to the treatment site while the prosthetic heart valve device moves between the containment configuration and the deployment configuration. Clinicians may thus position the sheathed prosthetic heart valve device at the desired target site for deployment, and then deploy the device at that target site without needing to compensate for any axial movement caused by deployment.

### SUMMARY

In percutaneous hydraulic delivery systems, prosthetic implants such as valves and scaffolds often require high levels of compaction within a delivery capsule to decrease the diameter and delivery profile of the delivery capsule and provide more atraumatic delivery through the vasculature of a patient. In some cases, this significant compaction of the prosthetic implant can lead to high deployment and recapture forces that may be difficult to deliver and control over long and flexible delivery systems, especially when navigating many turns within patient vasculature to reach a target implant or treatment site.

In some examples, the present disclosure is directed to hydraulically driven percutaneous prosthetic implant delivery systems and methods that include hydraulic tension and compression delivery systems that may or may not be independently pressurized. Independent tension and compression delivery systems and methods can withstand high deployment and recapture forces and may also enable flexibility in tracking or steering to a target implant site. Apparatuses and systems of the present disclosure may provide superior flexibility by utilizing a manifold that forms one or more independently isolated hydraulic circuits, each such hydraulic circuit having components that may be joined by a tensile member, which may be thin and flexible, and which may provide a mechanical connection of transmitted forces between multiple hydraulic circuits, where applicable.

In some examples, when hydraulic fluid is delivered to an inflation manifold of the device to pressurize a forward advancement fluid flow circuit, force is transmitted along an elongate lumen of a catheter to a distal tip of a delivery capsule with an internal bore, lumen, capsule, or other containment element configured to retain all or part of a prosthetic implant in a compressed state. The containment element of the delivery capsule is occupied by an implant advancement piston releasably attached to the prosthetic implant. Fluid is delivered through the implant advancement piston and into a chamber at a distal end of the delivery capsule, for example, which pushes the delivery capsule over the implant advancement piston in a distal direction with respect to the manifold and gradually unsheathes the implant. Once at least a portion of the prosthetic implant is free of the delivery capsule and exposed, the exposed portions of the prosthetic implant may begin to radially expand and deploy from the delivery capsule to, for example, engage tissue in or near the mitral valve annulus of the patient.

In some examples, a reverse fluid flow circuit that may be used for recapture or resheathing of all or part of the implant, as the case or need may be. When hydraulic fluid is delivered to the inflation manifold to pressurize the reverse fluid flow circuit, force is transmitted to an implant retraction piston moveable within a containment element of an proximal chamber in a handle of the device. The retraction piston may be mechanically joined to the delivery capsule by tensile member such as, for example, a metallic wire, a shaft, or a catheter. Force applied to the retraction piston by the fluid may thus move the retraction piston and the delivery capsule proximally toward the handle of the device, which retracts the delivery capsule and recaptures or re-sheathes all or part of the prosthetic implant device. In some examples, a biasing member such as a spring or adjustable push- or pull-wire assists movement of the retraction piston in the proximal direction. Since the retraction piston and the delivery capsule are mechanically connected, movement of the deployment capsule in the distal direction compresses the biasing member, and when the reverse fluid circuit is pressurized, the expansion of the biasing member further assists movement of the retraction piston in the proximal direction.

The reverse fluid flow circuit may operate independently of the advancement fluid flow circuit, such that only one hydraulic circuit is powered at any given time, while the alternate circuit is vented and operates only partly or not at all. Independently pressurizable hydraulic circuits may more precisely control deployment and recapture forces, which can improve the positioning of the implant in the mitral valve annulus of the patient and enhance the success rate of mitral valve replacement procedures.

In one aspect, the present disclosure is directed to an apparatus for hydraulic deployment and recapture of an implant. The apparatus includes a hydraulic implant uncovering mechanism and a hydraulic implant re-covering mechanism. The implant uncovering mechanism and the implant recovering mechanism are independently actuateable with a fluid, and the implant uncovering mechanism is coupled to the implant recovering mechanism by a flexible elongate tether.

In another aspect, the present disclosure is directed a hydraulic system for deployment and recapture of a prosthetic heart valve implant. The hydraulic system is connected to a source of pressurized fluid and includes a manifold assembly with a first portion that forms a advancement fluid flow circuit to hydraulically deploy the implant, and a second portion that forms a retraction fluid flow circuit to hydraulically recapture the implant. When the advancement fluid flow circuit is pressurized with fluid, the reverse fluid circuit is ventilated, and when the retraction fluid flow circuit is pressurized with fluid, the advancement fluid flow circuit is ventilated. A valve fluidly connected to the source of pressurized fluid and the hydraulic system selectably directs a flow of fluid between the advancement fluid flow circuit and the retraction fluid flow circuit.

In another aspect, the present disclosure is directed to a method for delivering a prosthetic heart implant to a native mitral valve of a heart of a human patient. The method includes: providing a hydraulic system connected to a source of pressurized fluid, wherein the hydraulic system includes a manifold assembly with a first portion that forms a advancement fluid flow circuit to hydraulically deploy the implant from the delivery capsule, and a second portion that forms a retraction fluid flow circuit to hydraulically recapture the implant, if the advancement fluid flow circuit is pressurized with fluid, the reverse fluid circuit is ventilated, and if the retraction fluid flow circuit is pressurized with fluid, the advancement fluid flow circuit is ventilated; positioning a delivery capsule of an elongated catheter body within the heart, the delivery capsule including a containment element having therein an advancement piston releasably attached to the prosthetic heart valve device; and directing a flow of fluid to the advancement fluid flow circuit of the hydraulic system to deliver fluid to a chamber in the delivery capsule to move the delivery capsule in a distal direction with respect to the advancement piston and unsheathe the prosthetic heart valve device from the delivery capsule to a deployment configuration in which the prosthetic heart valve device is at least partially radially expanded to engage tissue of the native mitral valve, and while fluid is delivered to the first chamber, draining fluid from the retraction fluid flow circuit.

In another aspect, the present disclosure is directed to an apparatus for hydraulic deployment and recapture of a prosthetic heart valve implant, the apparatus including a distal end with an implant deployment mechanism, a proximal end with an implant recapture mechanism, and a manifold assembly between the proximal end of the apparatus and the distal end of the apparatus, the manifold assembly including: a first portion that directs a fluid to the implant deployment mechanism of the apparatus, and a second portion that directs a fluid flow to the implant recapture mechanism of the apparatus; and a passage configured to allow proximal and distal translation of a tensile member that mechanically connects the implant deployment mechanism to the implant recapture mechanism.

Further disclosed herein is an apparatus for hydraulic deployment and recapture of an implant includes a hydraulic implant uncovering mechanism and a hydraulic implant recovering mechanism, wherein the implant uncovering mechanism and the implant re-covering mechanism are independently actuateable with a fluid, and the implant uncovering mechanism is coupled to the implant re-covering mechanism by a flexible elongate tether.

The details of one or more examples are set forth in the accompanying drawings and the description below. Other features, objects, and advantages of the invention will be apparent from the description and drawings, and from the claims.

### BRIEF DESCRIPTION OF DRAWINGS

FIG. 1 is a series of photographs schematically illustrating a transfemoral approach to placing a prosthetic heart valve implant.
FIG. 2 is a schematic, cut away view of an example of a system of the present disclosure.
FIG. 3A is a perspective view of an example of a delivery capsule of the system of FIG. 2.
FIG. 3B is a detail of a proximal edge of the delivery capsule of FIG. 3A.
FIG. 3C is a schematic, cut away view of an example of a delivery capsule in the system of FIG. 2.
FIG. 4 is a side view of an example of a prosthetic heart valve implant suitable for use in the system of FIG. 2.
FIG. 5 is a bottom view of an example of a prosthetic heart valve implant suitable for use in the system of FIG. 2.
FIG. 6A is a schematic, cut-away view of a recapture mechanism of the system of FIG. 2.
FIG. 6B is a schematic, cross-sectional view of the recapture mechanism of FIG. 6A.
FIG. 7 is a schematic, side view of a recapture mechanism of the system of FIG. 6A.
FIGS. 8, 9, 10, and 11 are schematic, cut-away views of successive steps of a method of delivering a prosthetic heart valve implant using the system of FIG. 2, showing the respective status of the components of the deployment mechanism and the recapture mechanism.
FIG. 12 is a system diagram showing the pressurized and vented states of the forward advancement fluid flow circuit and the reverse recapture fluid flow circuits of the system of FIG. 2.

Like symbols in the drawings indicate like elements.

### DETAILED DESCRIPTION

A mitral, tricuspid, aortic, or pulmonic, i.e., any atrioventricular valve, can be percutaneously accessed through the patient's vasculature. Depending on the point of vascular access, access to the mitral valve may be antegrade and may rely on entry into the left atrium by crossing the inter-atrial septum (e.g., a trans-septal approach, or TS). Alternatively, access to the valve can be retrograde, for example where the left ventricle is entered after passing through the aortic valve. Access to the mitral or other valve may also be achieved using a cannula via a trans-apical approach (TA).

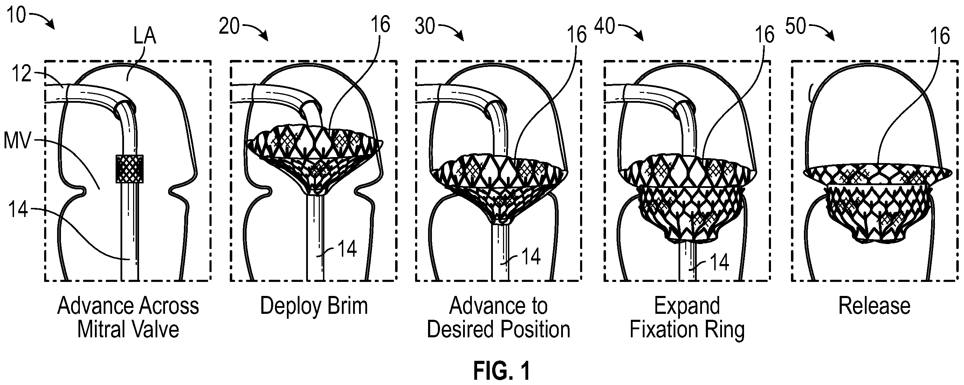

FIG. 1 shows a schematic summary of trans-septal or transfemoral approach to mitral valve replacement utilizing a prosthetic valve implant. In step 10, a catheter 12 is advanced into the left atrium LA above the mitral valve MV. A distal end of the catheter 12 includes a delivery capsule 14, which includes a prosthetic heart valve device compressed therein. As shown in step 10, the delivery capsule in advanced across the mitral valve MV. As shown in step 20, the delivery capsule 14 is hydraulically activated and translated in the distal direction with respect to a deployment handle (not shown in FIG. 1) used to guide the catheter 12. The distal movement of the delivery capsule 14 unsheathes the prosthetic device 16 and allows the prosthetic device to expand radially. In step 30, the catheter 12 may be utilized to advance the partially deployed prosthetic device 16, and in step 40 the delivery capsule 14 is further translated distally to unsheathe more of the prosthetic device 16 and place the prosthetic device 16 in the mitral valve septum. In step 50, the catheter 12 releases prosthetic device 16, and the delivery capsule 14 is withdrawn through the prosthetic device 16, leaving the prosthetic device in the target implant location in the heart of the patient.

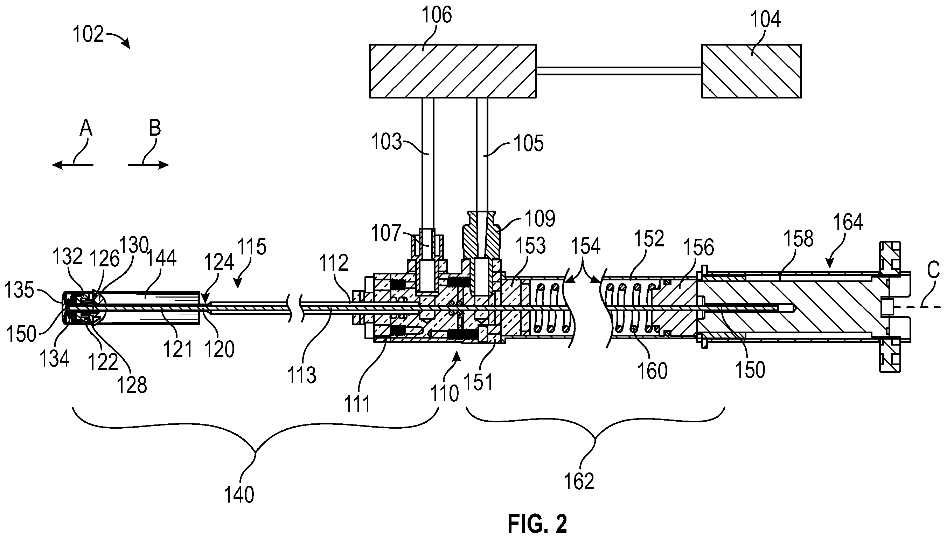

Referring now to FIG. 2, a hydraulic system 102 for deployment and recapture of a prosthetic heart valve device includes a fluid source 104 that supplies pressurized fluid to switchable valve system 106. In some examples, the fluid source 104 is a syringe or syringe-like fluid source, and in other examples it can include fully manual, partly manual, or fully or partly automated inflation devices, such as those available under the trade designation basixTOUCH 40 from Merit Medical Systems, Inc., South Jordan, UT. In one example, the fluid source 104 provides about 30 ml of a hydraulic fluid at a pressure of up to about 40 atm. A switchable valve system 106 directs the pressurized fluid from the fluid source 104 to either of two fluid lines 103, 105 connected to respective luer fittings 107, 109 on a fluid manifold 110.

The manifold 110 includes a first distal portion 111 connected to an elongate flexible catheter 112. As shown in FIG. 2, a fluid line 103 directs pressurized fluid through the distal portion 111 of the manifold 110 into a lumen 113 in the catheter 112. In some examples, the catheter 112 is steerable from a handle of the device 102, and may be used to help navigate the catheter 112 through vasculature or the heart of a patient. In some examples, the catheter 112 is within another steerable catheter or guide catheter (not shown in FIG. 2), which can further assist maneuverability of the catheter 112. In various examples, such catheters may be formed from a polymeric material, support or biasing strips or rings, tubes, a metal braid, or combinations thereof, or the like, and in some examples the lumen 113 may be lined with a material such as a polyimide to provide resilience under high hydraulic system pressures.

A distal end 115 of the catheter 112 includes a piston mount 120. In one example, the piston mount 120 may be attached to the catheter 112 by any suitable technique including, for example, an adhesive, or a mechanical attachment such as a crimp fitting. In another example, the piston mount 120 may be a portion of the catheter 112. The piston mount 120 includes a bore 121 that allows fluid to flow from the lumen 113 of the catheter 112 through the piston mount 120.

The piston mount 120 provides a support for a prosthetic heart valve implant (not shown in FIG. 2) that may be compressed entirely or, optionally, partly within delivery capsule 114. The piston mount 120 is rigid and extends through the compressed prosthetic heart valve implant. In some examples, the piston mount 120 may also provide column strength for the implant during deployment and recapture procedures. An implant advancement piston 122 is mounted on the piston mount 120 and resides in a bore 124 of the delivery capsule 114. The implant advancement piston 122 further includes at least one passage 126 that allows fluid to flow from the piston mount 120 through the piston 122. The implant advancement piston 122 further includes at least one mounting structure 128 such as, for example, a T-bar mount or slot, that releasably engages the prosthetic heart valve implant. A proximal surface of the implant advancement piston 122 may also include an optional compliant bumper 130. Upon release of the implant, the compliant bumper 130 is proximal of delivery capsule 114 and covers a proximal end of delivery capsule 114. In some examples, the bumper 130 provides an atraumatic surface that can prevent an edge of the delivery capsule 114 from catching and dislodging the leaflets of a prosthetic valve implant or damaging valve tissue or native patient anatomy.

The delivery capsule 114 further includes a distal chamber 132, which can be filled with pressurized fluid from the catheter 112 to move a distal end 135 of the delivery capsule 114 in a distal direction A relative to implant advancement piston 122. The distal end 135 of the delivery capsule 114 can have an atraumatic profile as shown in FIG. 2, or in some examples may include a pointed or dilating tip (not shown in FIG. 2). In some examples, the distal chamber 132 includes an optional biasing member such as a linear spring 134, which can assist movement of the delivery capsule 114 in moving along the distal direction A with respect to the implant advancement piston 122.

The fluid connections between the manifold 110, the catheter 112, the piston mount 120, and the implant advancement piston 122 provide a forward advancement fluid flow circuit 140 that delivers pressurized fluid into the distal chamber 132. As the distal chamber 132 fills with fluid, the delivery capsule 114 moves distally along direction A relative to implant advancement piston 122. As the delivery capsule moves distally, the compressed prosthetic heart valve implant therein is exposed and allowed to radially expand while remaining releasably attached to the implant advancement piston 122.

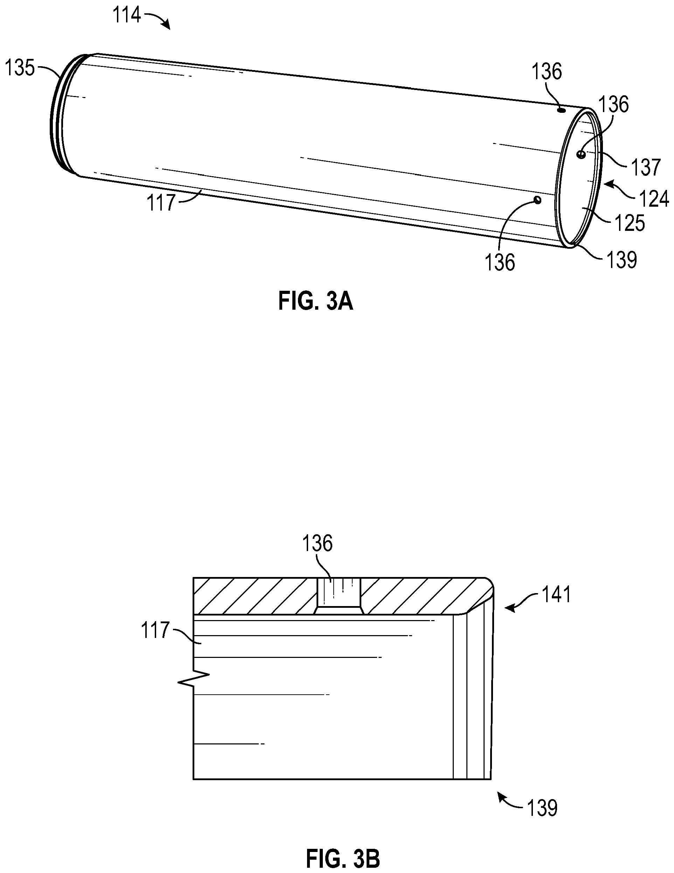

FIG. 3A provides a more detailed view of an example of the delivery capsule 114 of FIG. 2. The delivery capsule 114 includes a body 117 with the distal end 135 and a proximal end 137. The body 117 further includes a bore 124 that travels over the implant advancement piston 122. The bore 125 includes an internal surface 125 that may optionally be polished to provide a low friction interface with surfaces of the implant advancement piston 122. In some examples, the body 117 of the delivery capsule 114 may optionally include one or more vent apertures 136. The vent apertures 136 provide a pressure relief valve that can modulate predictable termination of the travel of the delivery capsule 114 in a proximal or a distal direction as the delivery capsule 114 moves over the implant advancement piston traversing the bore 124. For example, vent apertures 136 may be defined at a position along the length of delivery capsule 114 such that, when delivery capsule 114 and implant advancement piston 122 are positioned such that distal chamber 132 extends to vent apertures 136, the forces urging delivery capsule 114 distally (e.g., fluid pressure within distal chamber 132 and spring force from optional linear spring 134) are substantially balanced by forced urging delivery capsule 114 proximally (e.g., fluid pressure and/or spring force within a bore 154 (FIG. 2). This may reduce a likelihood that implant advancement piston 122 exits delivery capsule 114 upon releasing the implant and may assist with loading the implant to implant advancement piston 122.

FIG. 3B shows a view of a portion of a proximal edge 139 of the delivery capsule 114. In some examples, the proximal edge 139 can include an angled radius 141, which can reduce the force required to move the delivery capsule 114 in a proximal direction (along arrow B in FIG. 2).

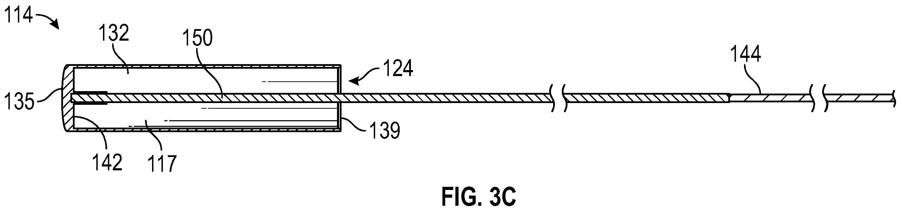

Referring to FIG. 3C, as well as FIG. 2, the body 117 of the delivery capsule 114 includes a distal internal wall 142 within the distal chamber 132 thereof. The distal internal wall 142 is attached to a tensile member 144, which mechanically connects the delivery capsule 114 to other components of the device 102. In some examples, the tensile member 144 is a metal cable, which translates within a jacket 150 to isolate the tensile member 144 from fluid flowing into or out of the distal chamber 132. The tensile member 144 can be attached to the distal internal wall 142 by any suitable technique including adhesives or mechanical attachments such as crimping and the like.

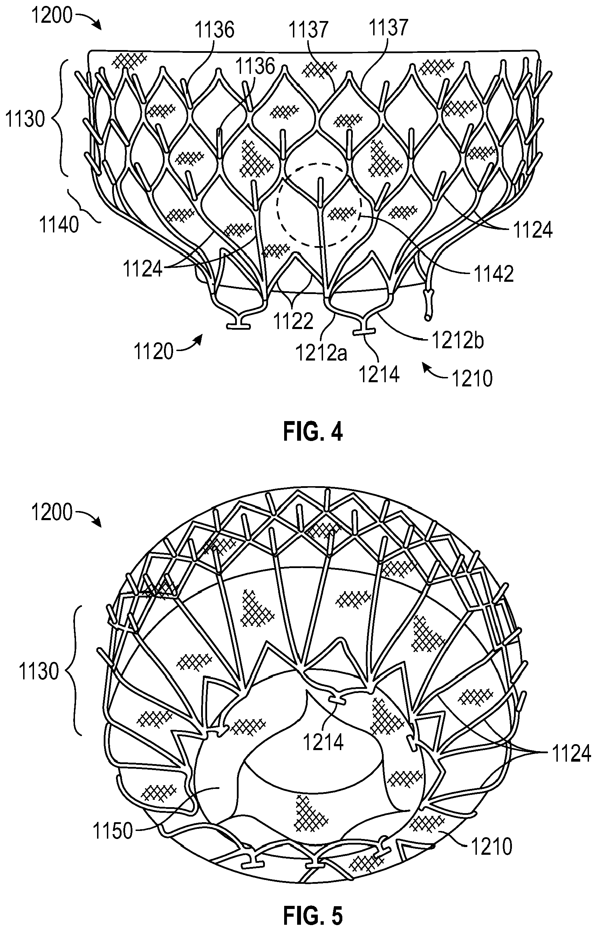

FIG. 4 is a side view and FIG. 5 is a bottom isometric view of an example embodiment, which is not intended to be limiting, of a prosthetic heart valve implant 1200 that may be compressed within the delivery capsule 114. In some examples, the implant 1200 may include additional members, which are not shown in FIGS. 4-5. Examples of other prosthetic heart valve implants are shown in US Published Patent Application 2018/0296325, which is incorporated herein by reference in its entirety.

In FIGS. 4-5, the implant 1200 includes an anchoring member 1120 with a base 1122 and a plurality of arms 1124 projecting laterally outward from the base 1122. The anchoring member 1120 also includes a fixation structure 1130 extending from the arms 1124. The fixation structure 1130 can be a ring (e.g., a straight cylinder, hourglass-shaped, or conical), and the outer surface of the fixation structure 1130 can define an annular engagement surface configured to press outwardly against a native annulus of a heart valve (e.g., a mitral valve). The fixation structure 1130 can further include a plurality of fixation elements 1136 that project radially outward and are inclined toward an upstream direction. The fixation elements 1136, for example, can be barbs, hooks, or other elements that are inclined only in the upstream direction (e.g., a direction extending away from the downstream portion of the device 1200). The fixation structure 1130 can also include structural elements 1137, which can be struts or other structural features formed from metal, polymers, or other suitable materials that can self-expand or be expanded by a balloon or other type of mechanical expander.

In some examples, the anchoring member 1120 has a smooth curve 1140 between the arms 1124 and the fixation structure 1130. The smooth curve 1140 is configured such that the implant 1200 can be recaptured or re-sheathed in a capsule or other container after the implant 1200 has been at least partially deployed.

The implant 1200 of this example embodiment includes connectors 1210 projecting from a base 1122 of the anchoring member 1120. The extended connectors 1210 can include a first strut 1212a attached to one portion of the base 1122 and a second strut 1212b attached to another portion of the base 1122. The first and second struts 1212a-b are configured to form a structure in which they extend toward each other in a downstream direction and are connected to each other at the bottom of the structure. The structure of the first and second struts 1212a-b causes the extension connector 1210 to elongate when the device 1200 is in a low-profile configuration within the delivery capsule 114 during delivery or partial deployment. The extended connectors 1210 further include an attachment element 1214 configured to releasably engage the slots 128 in the implant delivery piston 122 (FIG. 2). The attachment element 1214 can be a T-bar or other element that prevents the implant 1200 from being released from the delivery capsule 114 until desired. For example, a T-bar type attachment element 1214 can prevent the implant 1200 from moving axially during deployment or partial deployment until the delivery capsule 114 moves an sufficient distance in a distal direction to expose the slots 128 in the implant advancement piston 122. When the slots 128 are exposed, the attachment elements 1214 in the implant 1200 disengage and allow the implant 1200 to expand radially out of the deployment capsule 114 to allow for full deployment. In other examples, slots 128 can be substituted or supplemented with other retention and release means, such as mounting studs, clips, hooks, snaps, impressions, grooves, or other such structures and configurations.

Referring again to FIG. 2, the valve 106 can be switched to direct pressurized fluid via the fluid line 105 into a proximal portion 151 of the manifold 110. The proximal portion 151 of the manifold 110 includes a fluid outlet 153. An elongate generally tubular proximal chamber 152 is mounted to the proximal portion 151 of the manifold 110 and includes a bore 154 that receives fluid from the manifold 110 and the fluid outlet 153. A retraction piston 156 moves proximally and distally in the bore 154 along a longitudinal axis C of the device 102. The retraction piston 156 abuts a mechanical hardstop assembly 158 within a device handle assembly 164, which limits deployment motion of the retraction piston 156 (discussed in more detail below). The retraction piston 156 is attached to the tensile member 144 and the jacket 150 ( and as such is rigidly mechanically tethered to the deployment capsule 114.

The bore 154 of the proximal chamber 152 also includes an optional biasing member 160 such as, for example, a linear spring, which acts on a distal surface of the retraction piston 156.

The fluid connections between the manifold 110, the proximal chamber 152, and the recapture the retraction piston 156 provide a retraction fluid flow circuit 162. When pressurized fluid enters the fluid line 105, the proximal portion 151 of the manifold 110, and the proximal chamber 152, the fluid exerts force on the distal surface of the retraction piston 156 and moves the retraction piston in a proximal direction along arrow B in FIG. 2. Since the retraction piston 156 is mechanically attached to the delivery capsule 114 by the tensile member 144, proximal movement of the retraction piston 156 also moves the delivery capsule 114 in a proximal direction, which tends to recapture and re-sheathe the prosthetic implant in the bore 124 of the delivery capsule 114. Pressurizing the reverse fluid circuit provides a practitioner the option of re-sheathing the prosthetic implant at any point during an implant procedure, which can be used to adjust the positioning of the implant within the mitral valve septum (see for example, FIG. 1).

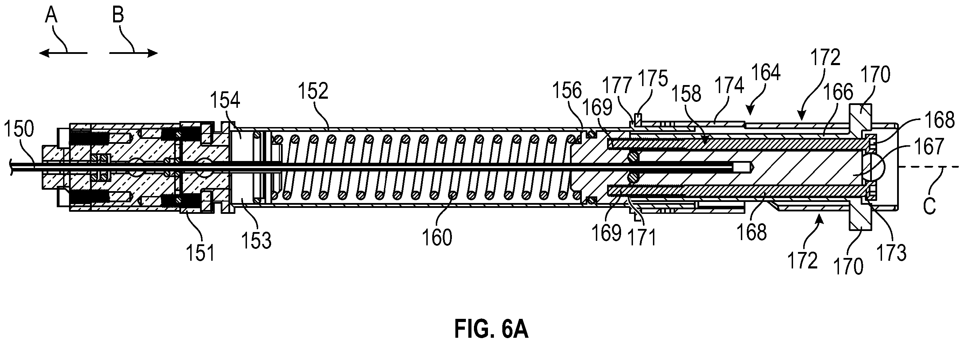

Referring now to FIGS. 6A, an example of a device handle 164 includes a mechanical hardstop assembly 158 that limits motion of the retraction piston 156 along a distal direction (arrow A in FIG. 2, reproduced in FIG. 6A). The hardstop assembly 158 includes a tubular stop member 166 with a bore 167.

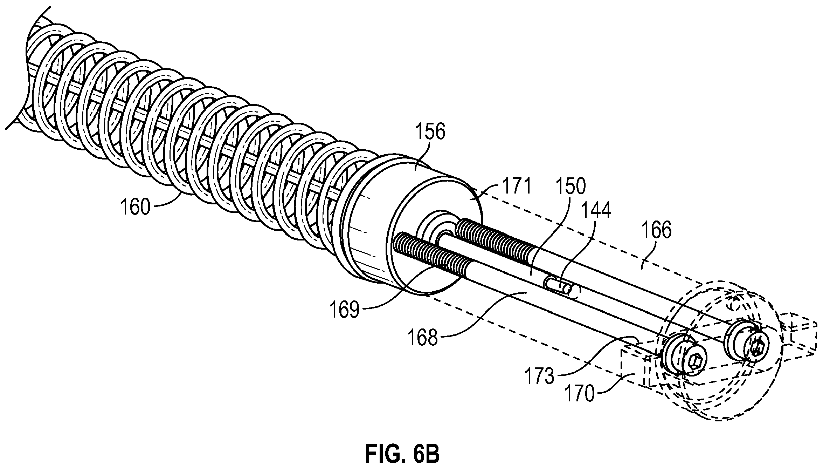

As also shown in FIG. 6B, the tubular stop member 166 is attached to retraction piston 156 by linear members such as the cross-sectioned screws 168 within the bore 167. A first end 169 of each screw 168 is affixed to a proximal face 171 of the retraction piston 156, and a second end 173 of each screw 168 is attached to the tubular stop member 166.

Referring to FIGS. 6A-6B, the tubular stop member 166 includes an arrangement of radially extending tabs 170 extending in a direction generally normal to a longitudinal axis C of the tubular stop member 166. The tubular stop member 166 lies within a rotatable collar 174. In the example of FIG. 6, the tabs 170 are configured to articulate within grooves 172 in the rotatable collar 174. The rotatable collar 174 includes an annular ridge 175, which abuts a corresponding annular ridge 177 on the proximal chamber 152.

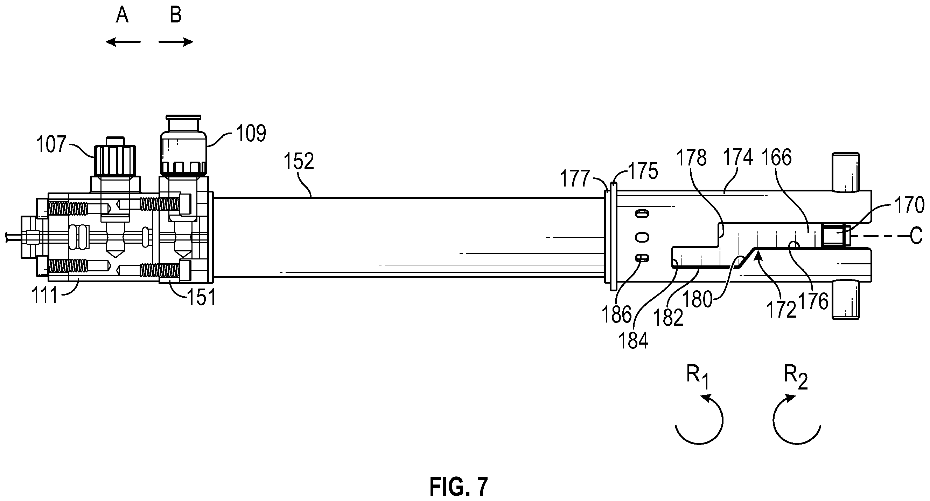

Referring also now to FIG. 7, an exterior view of the handle collar 174 includes an arrangement of grooves 172 that engage the tabs 170 on the tubular stop member 166. In the example of FIG. 7, the grooves 172 are staggered, and include a first linear section 176 terminated by a first ledge 178, an angled region 180, and a second linear section 182 terminated by a second ledge 184.

The collar 174 also includes an arrangement of slots 186 that engage with ball detents (not shown in FIG. 7) between the collar 174 and the underlying tubular stop member 166. In operation, the tab 170 on the tubular stop member 166 is within the first linear portion 176 of the groove 172 in the collar 174, and if the advancement fluid circuit 140 is pressurized, the distal chamber 132 of the delivery capsule 114 is filled with fluid and the distal tip 135 of the delivery capsule moves distally along the direction A in FIG. 7 (see also FIG. 2 for a general system overview). Since the reverse fluid circuit 162 is vented when the advancement fluid circuit is pressurized, the retraction piston 156, which is mechanically tethered to the delivery capsule 114 by the tensile member 144, also moves in the distal direction. The tubular stop member 166 is attached to the retraction piston 156, so as the retraction piston moves distally the tab 170 on the tubular stop member 166 also moves distally along the first linear portion of the groove 176. When the tab 170 engages the first ledge 178, the tubular stop member 166 arrests the distal movement of the retraction piston 156 and the delivery capsule 114 mechanically tethered thereto by the tensile member 144. This first ledge 178 thus provides an intermediate stop for the distal movement of the delivery capsule 114, and provides the practitioner an opportunity to adjust the placement of the catheter 112 or the delivery capsule 114 and its enclosed prosthetic implant. The first ledge 178 restrains the movement of the delivery capsule 114 in the distal direction, which can inhibit the radial expansion of the implant on the tissues of the patient during the deployment of the prosthetic implant.

When the collar 174 is rotated in the clockwise direction R2 about the longitudinal axis of the tubular stop member 166 (and the device 102, FIG. 2) an amount determined by the engagement of the ball detents with the slots 186, the tab 170 moves along the angled portion 180 of the staggered groove system 172 and enters the second linear portion 180 of the groove 172. Once the tab 170 enters the linear portion 182, the delivery capsule 114 can continue to move further in a distal direction, further deploying the prosthetic implant encapsulated therein. The second ledge 184 then arrests the distal motion of the tab 170, which in turn stops the distal movement of the delivery capsule 114, and acts as final limit on the distal travel of the delivery capsule 114.

If the reverse fluid circuit 162 is pressurized and the advancement fluid circuit is vented, fluid entering the proximal chamber 152 pushes the retraction piston 156 in a proximal direction along the direction of arrow B. The linear groove portion 182 in the rotatable collar 174 allows the tab 170 (and the tubular stop member 166) to freely move proximally until the tab 170 reaches the angled portion 180. When the tab 170 contacts the angled portion 180, the taper causes the slots 186 to disengage the ball detents and rotate collar 174 to allow continued proximal motion along first linear portion 176. The practitioner can use the angled portion 180 as a limiter for movement of the delivery capsule 114 in the proximal direction, or may rotate the collar 174 in the direction R2 to allow the tab 170 to move along the first linear portion 176 of the arrangement of grooves 172. When the tab 170 contacts the angled portion 180, the angled portion 180 causes the slots 186 to disengage the ball detents and rotate collar 174 to allow continued proximal motion along first linear portion 176.

Although FIG. 7 shows an arrangement of grooves 172 with two linear sections and two ledges, other arrangements such as, for example, a three-or-more tiered sequence of linear sections, may be used.

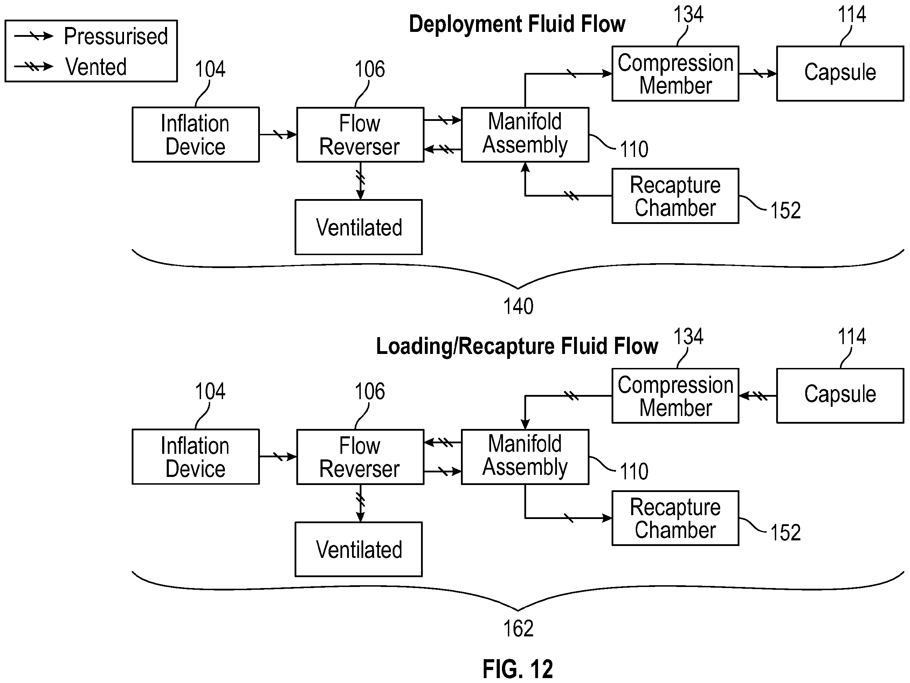

Referring now to the schematic diagram in FIG. 12, the advancement fluid flow circuit 140 and the reverse or retraction fluid flow circuit 162 (FIG. 2) are configured to operate independently of each other in this example. As shown in FIG. 12, when the advancement fluid flow circuit 140 is pressurized and activated (single hash marks), the retraction fluid flow circuit 162 is vented (dual hash marks). In the advancement fluid flow circuit 140, the inflation device 104 supplies fluid to a flow reversing valve 106, which in turn supplies fluid to the distal portion 111 of the manifold 110. The fluid then moves along the lumen 113 in the catheter 112 and enters the distal chamber 132 in the delivery capsule 114. Assisted by the compression spring 134 in this and other examples that employ such a spring, the delivery capsule 114 moves in a distal direction with respect to the manifold 110 and the handle 164 (FIG. 2) to begin deploying or unsheathing the prosthetic implant compressively housed therein. The distal movement of the delivery capsule 114 along the distal direction A in FIG. 2 causes the retraction piston 156 (which is mechanically linked to the delivery capsule 114 by the tensile member 144) to move in the distal direction, which in turn compresses the biasing member 160.

If the practitioner then switches the fluid flow in the flow reversing valve 106 to activate and pressurize the retraction fluid flow circuit 162, fluid moves into the proximal chamber 152 and exerts force on the retraction piston 154. Since the retraction piston is mechanically tethered to the delivery capsule 114 by the tensile member 144, the biasing member 160 in the proximal chamber 152 then works with the fluid pressure in the proximal chamber 152 to move the retraction piston 154 in a proximal direction, which also moves the delivery capsule 114 in a proximal direction to re-sheathe or recapture the implant stored therein.

In another aspect, the present disclosure is also directed to a method for delivering a prosthetic heart implant to a native mitral valve of a heart of a human patient. In this method a flexible and steerable catheter with a delivery capsule on a distal end thereof is translated through the vasculature of a patient (see example approach outlined in FIG. 1 above).

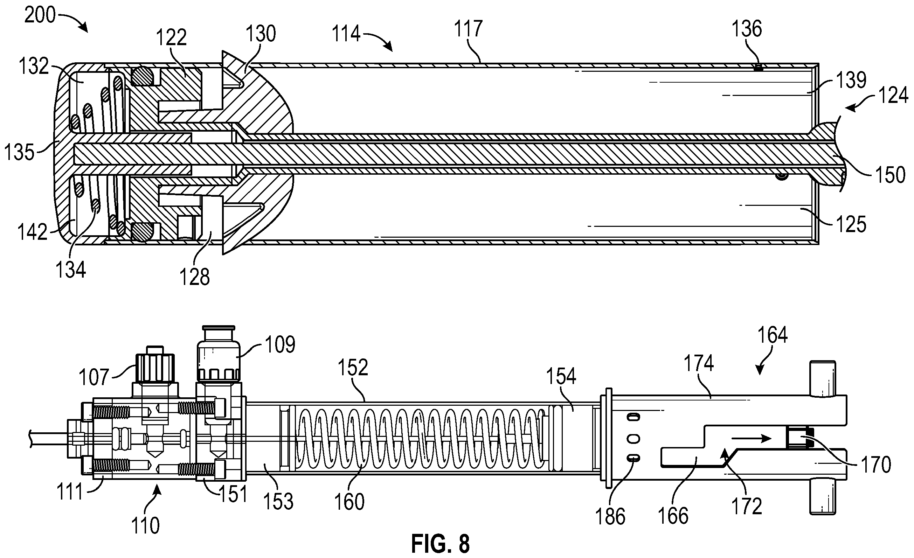

In the operation of this method, as shown schematically in FIG. 8, in a first step 200, the advancement piston 122 is in a distal position such that the distal compression spring 134 is fully compressed against the distal internal wall 142 of the body 117 of the delivery capsule 114. The prosthetic implant (not shown in FIGS. 8-11) is compressed and loaded in the internal bore 124 of the delivery capsule 114.

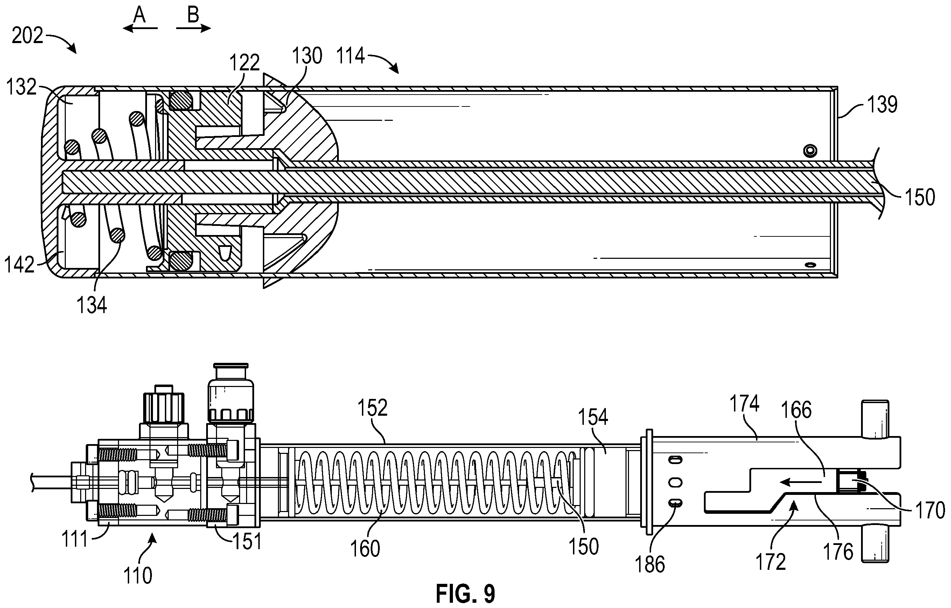

In a second step 202 shown schematically in FIG. 9, the advancement fluid flow circuit 140 (FIG. 2) is pressurized, which allows fluid to flow through the catheter 112 (not shown in FIG. 9) into the distal chamber 132 in the delivery capsule 114. The fluid pressure on the distal wall 142 of the delivery capsule 114, along with the force exerted by the distal compression spring 134, moves the delivery capsule 114 in a distal direction A with respect to the advancement piston 122. The prosthetic implant attached to the advancement piston 122 by the T-bar slots 128 (not shown in FIG. 9) then begins to deploy and radially expand (See, for example, FIG. 1.). Since the delivery capsule 114 is mechanically tethered to the retraction piston 154 by the tensile member 144, and the retraction fluid flow circuit is vented, the distal movement of the delivery capsule 114 moves the retraction piston 154 in a distal direction, compressing the spring 160 in the proximal chamber 152. The distal movement of the recapture spring 154 also moves the tubular stop member 166 in the distal direction, and the tab 170 thereon also moves distally and enters the first linear groove portion 176.

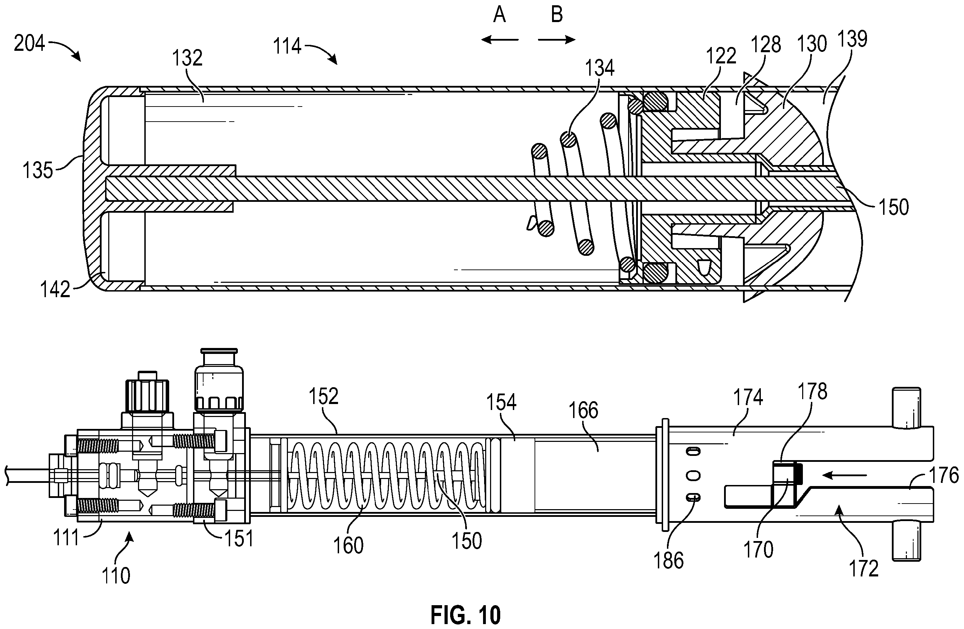

Referring now to step 204 illustrated schematically in FIG. 10, as the advancement fluid flow circuit remains open and pressurized, additional fluid enters the distal chamber 132 of the delivery capsule 114, which exerts additional force on the distal wall 142 thereof. The pressure exerted by the fluid pushes the delivery capsule 114 further in a distal direction along arrow A, further deploying the prosthetic implant. Due to the mechanical connection between the delivery capsule 114 and the retraction piston 154 via the tensile member 144, further distal movement of the delivery capsule 114 further compresses the recapture spring 160. The distal movement of the recapture spring 154 also moves the tubular stop member 166 distally until the tab 170 thereon engages the first ledge 178 in the system of grooves 172. The first ledge 178 in the collar 174 thus forms a hardstop that limits further advancement of the delivery capsule 114 in the distal direction and allows the practitioner to evaluate at least one of the functionality or the positioning of the gradually deploying If the practitioner has concerns about the functionality or positioning of the implant, the retraction fluid flow circuit could be pressurized and the advancement fluid flow circuit vented. Fluid flowing into the proximal chamber 152 then exerts force against the retraction piston 154, and recapture spring 160 elongates to push the tab 170 proximally along the direction of the arrow B within the first linear groove portion 176.

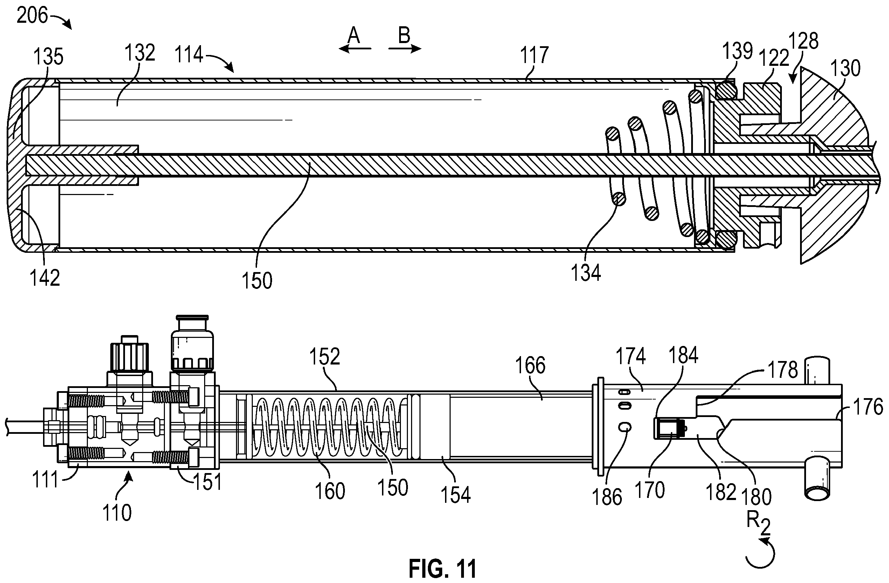

Referring now to step 206 in FIG. 11, with the advancement fluid flow circuit activated and the retraction fluid flow circuit vented, additional fluid fills the chamber 132 of the deployment capsule 114 and pushes the deployment capsule 114 farther along a distal direction (arrow A). Since the deployment capsule is mechanically connected to the retraction piston 156, the retraction piston 156 and the tubular stop member 166 also move distally, even further compressing the recapture spring 160. Rotation of the collar 174 in the clockwise direction R2 allows the tab 170 on the tubular stop member 166 to bypass the first ledge 178, move along the angled portion 180, and enter the second linear groove portion 182. The tab 170 moves distally along the second linear groove portion 182 until the motion of the tab 170 is arrested by the second ledge 184. In some examples, the delivery capsule 114 vents prior to the tab 170 contacting the second ledge 184, but in such cases the second ledge 184 can still limit motion if the venting of the delivery capsule 114 is insufficient. When the tab 170 reaches the second ledge 184, the delivery capsule 114 has moved a sufficient distance distally that the advancement piston 122 partially emerges from the proximal end 139 of the body 117 of the delivery capsule 114. The T-bar slots 128 are then exposed and free of the proximal end 139 of the delivery capsule 114, which releases the tabs 1214 (FIGS. 4-5) on the prosthetic implant, which is now properly seated in the mitral valve septum (FIG. 1).

The delivery capsule 114 may then be withdrawn from the seated prosthetic implant and, and then further withdrawn from the vasculature of the patient (not shown in FIG. 11).

The systems, apparatus and methods of the present disclosure are well suited to treat heart valves of the body, such as the mitral valve, and provide for repositioning and removal of a partially deployed prosthetic implant device. While the apparatus and methods are particularly well-suited for trans-septal and trans-apical approaches, the techniques described herein can also be adapted for trans-atrial and direct aortic delivery of a prosthetic replacement valve to a target location in the heart. The examples of the systems, apparatus and method of the present disclosure can be combined with many known surgeries and procedures, such as known methods of accessing the valves of the heart (e.g., the mitral valve or triscuspid valve) with antegrade or retrograde approaches, and combinations thereof.

The systems and methods described herein facilitate controlled delivery of a prosthetic heart valve implant using trans-apical or trans-septal delivery approaches and allow resheathing of the prosthetic heart valve device after partial deployment of the device to reposition and/or remove the device. In some examples, the independently pressurizable advancement fluid flow and retraction fluid flow circuits can be switchably filled with fluid and vented to initiate deployment and resheathing of the prosthetic device. The independent fluid flow circuits can facilitate more precise hydraulic control and power for accurate proximal and distal movement of the delivery capsule housing, which can more precisely control the delivery of the prosthetic heart valve implant.

The disclosure herein includes the following examples:
Example 1. An apparatus for hydraulic deployment and recapture of an implant, the apparatus comprising:
   a hydraulic implant uncovering mechanism, and
   a hydraulic implant re-covering mechanism,
   wherein the implant uncovering mechanism and the implant re-covering mechanism are independently actuateable with a fluid, and wherein the implant uncovering mechanism is coupled to the implant re-covering mechanism by a flexible elongate tether.
Example 2. The apparatus of Example 1, wherein the implant uncovering mechanism comprises a capsule having a side wall, an end wall, and an open end.
Example 3. The apparatus of Examples 1 or 2, wherein the capsule further comprises an internal chamber defined by a length of the side wall, the end wall, and an advancement piston comprising a first advancement-piston surface that faces the end wall and second advancement-piston surface that faces away from the first advancement-piston surface.
Example 4. The apparatus of Example 3, wherein the advancement piston is longitudinally slidable along the side wall.
Example 5. The apparatus of Examples 3 or 4, wherein the implant uncovering mechanism further comprises a fluid passage passing through the piston from the plane of the first surface to the plane of the second surface.
Example 6. The apparatus of Example 5, wherein the elongate tether passes through the advancement piston and is coupled to the implant uncovering mechanism at the end wall.
Example 7. The apparatus of any of Examples 1 to 6, wherein the implant re-covering mechanism comprises a proximal chamber having a sealed distal end.
Example 8. The apparatus of any of Examples 2 to 7, wherein the implant re-covering mechanism comprises a retraction piston comprising a first retraction-piston surface that faces the end wall and a second retraction-piston surface that faces away from the second retraction-piston surface.
Example 9. The apparatus of Example 8, wherein the first retraction-piston surface abuts a biasing member.
Example 10. The apparatus of Example 9, wherein the biasing member is configured to urge the capsule toward the distal end of the apparatus.
Example 11. The apparatus of Example 10, wherein the biasing member in the delivery capsule comprises a spring.
Example 12. The apparatus of any of Examples 8 to 11, wherein the second retraction-piston surface abuts a stop mechanism configured to limit travel of the retraction piston toward the end wall.
Example 13. The apparatus of any of Examples 3 to 12, wherein the implant-advancement piston comprises implant-retention slots.
Example 14. The apparatus of any of Examples 2 to 13, wherein the capsule side wall comprises an arrangement of vent apertures.
Example 15. The apparatus of any of Examples 11 to 14, wherein the spring is a linear spring.
Example 16. The apparatus of any of Examples 1 to 15, wherein the tensile member comprises a metal cable.
Example 17. A hydraulic system for hydraulic deployment and recapture of a prosthetic heart valve implant, wherein the hydraulic system is connected to a fluid source, the system comprising:
   a manifold assembly comprising a first portion that forms a advancement fluid flow circuit to hydraulically deploy the implant, and a second portion that forms a retraction fluid flow circuit to hydraulically recapture the implant, and when the advancement fluid flow circuit is pressurized with fluid, the reverse fluid circuit is ventilated, and when the retraction fluid flow circuit is pressurized with fluid, the advancement fluid flow circuit is ventilated; and
   a valve fluidly connected to the source of pressurized fluid and the hydraulic system, wherein the valve selectably directs a flow of fluid between the advancement fluid flow circuit and the retraction fluid flow circuit.
Example 18. The system of Example 17, wherein the manifold assembly comprises a distal end with an implant deployment mechanism fluidly connected to the advancement fluid flow circuit, and a proximal end with an implant recapture mechanism fluidly connected to the retraction fluid flow circuit.
Example 19. The system of Examples 17 or 18, wherein the implant deployment mechanism comprises an elongate flexible catheter attached to the first portion of the manifold, wherein the catheter comprises an internal lumen in fluid communication with a distal outlet of the manifold, an implant advancement piston mounted to the flexible catheter, and a delivery capsule a capsule configured to house the implant, wherein delivery capsule comprises an internal containment element that engages the implant advancement piston, and wherein a reversible flow of the fluid from the lumen of the catheter into a distal chamber of the delivery capsule urges a distal tip of the capsule toward the distal end of the apparatus such that the delivery capsule moves slidably over the implant advancement piston.
Example 20. The system of any of Examples 17 to 19, wherein the implant recapture mechanism comprises an elongate tubular proximal chamber attached to the second portion of the manifold, wherein the proximal chamber comprises an internal passage in fluid communication with a proximal outlet of the manifold, the proximal chamber comprising an internal containment element with an implant retraction piston therein, an wherein flow of the pressurized fluid into the proximal chamber urges the implant retraction piston toward the proximal end of the proximal chamber.
Example 21. The system of Example 20, wherein a distal end of the retraction piston abuts a linear spring and proximal end of the implant retraction piston abuts a stop mechanism configured to limit travel of the implant retraction piston within the containment element of the proximal chamber and toward the proximal end of the apparatus.
Example 22. The system of Examples 20 or 21, wherein a tensile member mechanically tethers the delivery capsule to the implant retraction piston such that the capsule and the implant retraction piston move in the same direction.
Example 23. The system of Example 22, wherein pressurized fluid flow into the implant deployment mechanism moves the delivery capsule toward a distal end of the apparatus and biases the implant retraction piston against the linear spring, and wherein pressurized fluid flow into the implant recapture mechanism urges the implant retraction piston in a proximal direction.
Example 24. The system of any of Examples 19 to 23, wherein the distal chamber of the delivery capsule comprises a spring that urges the delivery capsule toward the distal end of the apparatus and away from the implant advancement piston.
Example 25. The system of any of Examples 19 to 24, wherein a proximal end of the implant advancement piston comprises a compliant bumper.
Example 26. The system of any of Examples 19 to 25, wherein a proximal end of the implant advancement piston comprises an arrangement of slots that configured to releasably retain an arrangement of corresponding tabs on the prosthetic implant.
Example 27. The system of any of Examples 19 to 26, wherein a wall of the delivery capsule comprises an arrangement of vent apertures.
Example 28. The system of any of Examples 21 to 27, wherein the stop mechanism comprises an arrangement of linear rods affixed to the implant retraction piston and extending toward a proximal end of the apparatus.
Example 29. The system of Example 28, wherein the rods are retained within a tubular stop member.
Example 30. The system of Example 29, wherein the tubular stop member comprises at least one radially extending tab that extends in a direction substantially normal to a longitudinal axis of the apparatus.
Example 31. The system of Example 30, wherein at least a portion of an outer surface of a body of the implant proximal chamber is overlain by a collar, and wherein the collar is rotatable about a longitudinal axis of the apparatus.
Example 32. The system of Example 31, wherein the body of the collar comprises a staggered groove that engages the tabs on the tubular stop member, wherein the staggered groove in the collar comprises an arrangement of ledges that provide stops for the tabs on the tubular stop member.
Example 33. The system of Example 32, wherein rotation of the collar disengages the tabs on the tubular stop member from each ledge in the arrangement of ledges.
Example 34. The system of Example 33, wherein the staggered groove in the collar comprises an angled region to guide the tabs on the tubular stop member from a first ledge in the arrangement of ledges to a second edge in the arrangement of ledges as the collar is rotated from a first position to a second position.
Example 35. The system of Example 34, wherein the collar comprises an arrangement of slots, and wherein the slots engage ball detents on the outer surface of the implant proximal chamber to provide rotational tactile feedback to guide the tabs on the tubular stop member from a first ledge in the arrangement of ledges to a second edge in the arrangement of ledges as the collar is rotated from a first position to a second position.
Example 36. The system of any of Examples 19 to 35, further comprising a steerable catheter overlying the elongate flexible catheter.
Example 37. The system of any of Examples 19 to 35, wherein the elongate flexible catheter is steerable.
Example 38. The system of Example 37, further comprising an introducer sheath.
Example 39. The system of any of Examples 17 to 38, wherein the source of pressurized fluid comprises a pump.
Example 40. The system of any of Examples 17 to 39, wherein the source of pressurized fluid comprises a syringe.
Example 41. The system of any of Examples 17 to 40, wherein the valve comprises a switchable flow reverser.
Example 42. A method for delivering a prosthetic heart implant to a native mitral valve of a heart of a human patient, the method comprising:
   providing a hydraulic system connected to a source of pressurized fluid, wherein the hydraulic system comprises a manifold assembly comprising a first portion that forms a advancement fluid flow circuit to hydraulically deploy the implant from the delivery capsule, and a second portion that forms a retraction fluid flow circuit to hydraulically recapture the implant, if the advancement fluid flow circuit is pressurized with fluid, the reverse fluid circuit is ventilated, and if the retraction fluid flow circuit is pressurized with fluid, the advancement fluid flow circuit is ventilated;
   positioning a delivery capsule of an elongated catheter body within the heart, the delivery capsule comprising a containment element having therein a advancement piston releasably attached to the prosthetic heart valve device; and
   directing a flow of fluid to the advancement fluid flow circuit of the hydraulic system to deliver fluid to a chamber in the delivery capsule to move the delivery capsule in a distal direction with respect to the advancement piston and unsheathe the prosthetic heart valve device from the delivery capsule to a deployment configuration in which the prosthetic heart valve device is at least partially radially expanded to engage tissue of the native mitral valve, and
   while fluid is delivered to the first chamber, draining fluid from the retraction fluid flow circuit.
Example 43. The method of Example 42, further comprising:
   after allowing the prosthetic heart device to at least partially radially expand out of the delivery capsule, directing a flow of fluid to the retraction fluid flow circuit to move a retraction piston mechanically tethered to the delivery capsule, wherein the retraction piston moves the delivery capsule to re-sheathe the prosthetic heart valve device within the delivery capsule and disengage the tissue of the native mitral valve; and
   while fluid is delivered to the retraction fluid flow circuit, draining fluid from the advancement fluid flow circuit.
Example 44. The method of Examples 42 or 43, further comprising moving the delivery capsule in the distal direction with a spring biased against the implant advancement piston.
Example 45. The method of any of Examples 42 to 44, further comprising damping movement of the delivery capsule in the delivery capsule with an arrangement of ventilation holes in a body of the delivery capsule.
Example 46. The method of any of Examples 43 to 45, further comprising moving the retraction piston in a proximal direction with a spring biased against the retraction piston.
Example 47. The method of any of Examples 43 to 46, wherein motion of the retraction piston in a proximal direction is limited by a mechanical stop attached to the retraction piston.
Example 48. The method of Example 47, wherein the stop mechanism comprises an arrangement of linear rods affixed to the implant retraction piston, and wherein the rods are retained within a tubular stop member.
Example 49. The method of Example 48, wherein at least a portion of an outer surface of a body of the implant proximal chamber is overlain by a collar, and wherein the body of the collar comprises a staggered groove that engages an arrangement of tabs on the tubular stop member.
Example 50. The method of Example 49, wherein the collar comprises an arrangement of slots, and wherein the slots engage ball detents on the outer surface of the implant proximal chamber to provide rotational tactile feedback to guide the tabs on the tubular stop member.
Example 51. An apparatus for hydraulic deployment and recapture of a prosthetic heart valve implant, the apparatus comprising a distal end with an implant deployment mechanism, a proximal end with an implant recapture mechanism, and a manifold assembly between the proximal end of the apparatus and the distal end of the apparatus,
   the manifold assembly comprising:
   a first portion that directs a fluid to the implant deployment mechanism of the apparatus, and
   a second portion that directs a fluid flow to the implant recapture mechanism of the apparatus; and
   a passage configured to allow proximal and distal translation of a tensile member that mechanically connects the implant deployment mechanism to the implant recapture mechanism.

Various examples have been described. These and other examples are within the scope of the following claims.

Further disclosed herein is the subject-matter of the following clauses:
1. An apparatus for hydraulic deployment and recapture of an implant, the apparatus comprising:
   a hydraulic implant uncovering mechanism, and
   a hydraulic implant re-covering mechanism,
   wherein the implant uncovering mechanism and the implant re-covering mechanism are independently actuateable with a fluid, and wherein the implant uncovering mechanism is coupled to the implant re-covering mechanism by a flexible elongate tether.
2. The apparatus of clause 1, wherein the implant uncovering mechanism comprises a capsule having a side wall, an end wall, and an open end, and wherein the capsule further comprises an internal chamber defined by a length of the side wall, the end wall, and an advancement piston comprising a first advancement-piston surface that faces the end wall and second advancement-piston surface that faces away from the first advancement-piston surface.
3. The apparatus of clause 1 or of any of the preceding clauses, wherein the implant uncovering mechanism further comprises a fluid passage passing through the piston from the plane of the first surface to the plane of the second surface.
4. The apparatus of clause 3, wherein the elongate tether passes through the advancement piston and is coupled to the implant uncovering mechanism at the end wall.
5. The apparatus of clause 1 or of any of the preceding clauses, wherein the implant recovering mechanism comprises a proximal chamber having a sealed distal end, and wherein the implant re-covering mechanism comprises a retraction piston comprising a first retraction-piston surface that faces the end wall and a second retraction-piston surface that faces away from the second retraction-piston surface.
6. The apparatus of clause 5, wherein the first retraction-piston surface abuts a biasing member configured to urge the capsule toward the distal end of the apparatus.
7. The apparatus of clause 5, wherein the second retraction-piston surface abuts a stop mechanism configured to limit travel of the retraction piston toward the end wall.
8. A hydraulic system for hydraulic deployment and recapture of a prosthetic heart valve implant, wherein the hydraulic system is connected to a fluid source, the system comprising:
   a manifold assembly comprising a first portion that forms a advancement fluid flow circuit to hydraulically deploy the implant, and a second portion that forms a retraction fluid flow circuit to hydraulically recapture the implant, and when the advancement fluid flow circuit is pressurized with fluid, the reverse fluid circuit is ventilated, and when the retraction fluid flow circuit is pressurized with fluid, the advancement fluid flow circuit is ventilated; and
   a valve fluidly connected to the source of pressurized fluid and the hydraulic system, wherein the valve selectably directs a flow of fluid between the advancement fluid flow circuit and the retraction fluid flow circuit.
9. The system of clause 8, wherein the manifold assembly comprises a distal end with an implant deployment mechanism fluidly connected to the advancement fluid flow circuit, and a proximal end with an implant recapture mechanism fluidly connected to the retraction fluid flow circuit.
10. The system of clause 8 or of any of clauses 8-9, wherein the implant deployment mechanism comprises an elongate flexible catheter attached to the first portion of the manifold, wherein the catheter comprises an internal lumen in fluid communication with a distal outlet of the manifold, an implant advancement piston mounted to the flexible catheter, and a delivery capsule a capsule configured to house the implant, wherein delivery capsule comprises an internal containment element that engages the implant advancement piston, and wherein a reversible flow of the fluid from the lumen of the catheter into a distal chamber of the delivery capsule urges a distal tip of the capsule toward the distal end of the apparatus such that the delivery capsule moves slidably over the implant advancement piston.
11. The system of clause 8 or of any of clauses 8-10, wherein the implant recapture mechanism comprises an elongate tubular proximal chamber attached to the second portion of the manifold, wherein the proximal chamber comprises an internal passage in fluid communication with a proximal outlet of the manifold, the proximal chamber comprising an internal containment element with an implant retraction piston therein, an wherein flow of the pressurized fluid into the proximal chamber urges the implant retraction piston toward the proximal end of the proximal chamber.
12. The system of clause 11, wherein a distal end of the retraction piston abuts a linear spring and proximal end of the implant retraction piston abuts a stop mechanism configured to limit travel of the implant retraction piston within the containment element of the proximal chamber and toward the proximal end of the apparatus.
13. The system of clause 8 or of any of clauses 8-12, wherein a tensile member mechanically tethers the delivery capsule to the implant retraction piston such that the capsule and the implant retraction piston move in the same direction.
14. The system of clause 13, wherein pressurized fluid flow into the implant deployment mechanism moves the delivery capsule toward a distal end of the apparatus and biases the implant retraction piston against the linear spring, and wherein pressurized fluid flow into the implant recapture mechanism urges the implant retraction piston in a proximal direction.
15. The system of clause 14, wherein the distal chamber of the delivery capsule comprises a spring that urges the delivery capsule toward the distal end of the apparatus and away from the implant advancement piston.
16. A method for delivering a prosthetic heart implant to a native mitral valve of a heart of a human patient, the method comprising:
   providing a hydraulic system connected to a source of pressurized fluid, wherein the hydraulic system comprises a manifold assembly comprising a first portion that forms a advancement fluid flow circuit to hydraulically deploy the implant from the delivery capsule, and a second portion that forms a retraction fluid flow circuit to hydraulically recapture the implant, if the advancement fluid flow circuit is pressurized with fluid, the reverse fluid circuit is ventilated, and if the retraction fluid flow circuit is pressurized with fluid, the advancement fluid flow circuit is ventilated;
   positioning a delivery capsule of an elongated catheter body within the heart, the delivery capsule comprising a containment element having therein a advancement piston releasably attached to the prosthetic heart valve device; and
   directing a flow of fluid to the advancement fluid flow circuit of the hydraulic system to deliver fluid to a chamber in the delivery capsule to move the delivery capsule in a distal direction with respect to the advancement piston and unsheathe the prosthetic heart valve device from the delivery capsule to a deployment configuration in which the prosthetic heart valve device is at least partially radially expanded to engage tissue of the native mitral valve, and
   while fluid is delivered to the first chamber, draining fluid from the retraction fluid flow circuit.
17. The method of clause 16, further comprising:
   after allowing the prosthetic heart device to at least partially radially expand out of the delivery capsule, directing a flow of fluid to the retraction fluid flow circuit to move a retraction piston mechanically tethered to the delivery capsule, wherein the retraction piston moves the delivery capsule to re-sheathe the prosthetic heart valve device within the delivery capsule and disengage the tissue of the native mitral valve; and
   while fluid is delivered to the retraction fluid flow circuit, draining fluid from the advancement fluid flow circuit.
18. The method of clause 16, further comprising moving the delivery capsule in the distal direction with a spring biased against the implant advancement piston.
19. The method of clause 18, further comprising damping movement of the delivery capsule in the delivery capsule with an arrangement of ventilation holes in a body of the delivery capsule.
20. The method of clause 16, further comprising moving the retraction piston in a proximal direction with a spring biased against the retraction piston.

## Claims

1. An apparatus for hydraulic deployment and recapture of an implant, the apparatus comprising:
a hydraulic implant uncovering mechanism, and
a hydraulic implant re-covering mechanism,
wherein the implant uncovering mechanism and the implant re-covering mechanism are independently actuateable with a fluid, and wherein the implant uncovering mechanism is coupled to the implant re-covering mechanism by a flexible elongate tether.

2. The apparatus of claim 1, wherein the implant uncovering mechanism comprises a capsule having a side wall, an end wall, and an open end, and wherein the capsule further comprises an internal chamber defined by a length of the side wall, the end wall, and an advancement piston comprising a first advancement-piston surface that faces the end wall and second advancement-piston surface that faces away from the first advancement-piston surface.

3. The apparatus of any of the preceding claims, wherein the implant uncovering mechanism further comprises a fluid passage passing through the piston from the plane of the first surface to the plane of the second surface.

4. The apparatus of claim 3, wherein the elongate tether passes through the advancement piston and is coupled to the implant uncovering mechanism at the end wall.

5. The apparatus of any of the preceding claims, wherein the implant re-covering mechanism comprises a proximal chamber having a sealed distal end, and wherein the implant re-covering mechanism comprises a retraction piston comprising a first retraction-piston surface that faces the end wall and a second retraction-piston surface that faces away from the second retraction-piston surface.

6. The apparatus of claim 5, wherein the first retraction-piston surface abuts a biasing member configured to urge the capsule toward the distal end of the apparatus.

7. The apparatus of claim 5, wherein the second retraction-piston surface abuts a stop mechanism configured to limit travel of the retraction piston toward the end wall.

8. A hydraulic system for hydraulic deployment and recapture of a prosthetic heart valve implant, wherein the hydraulic system is connected to a fluid source, the system comprising:
a manifold assembly comprising a first portion that forms a advancement fluid flow circuit to hydraulically deploy the implant, and a second portion that forms a retraction fluid flow circuit to hydraulically recapture the implant, and when the advancement fluid flow circuit is pressurized with fluid, the reverse fluid circuit is ventilated, and when the retraction fluid flow circuit is pressurized with fluid, the advancement fluid flow circuit is ventilated; and
a valve fluidly connected to the source of pressurized fluid and the hydraulic system, wherein the valve selectably directs a flow of fluid between the advancement fluid flow circuit and the retraction fluid flow circuit.

9. The system of claim 8, wherein the manifold assembly comprises a distal end with an implant deployment mechanism fluidly connected to the advancement fluid flow circuit, and a proximal end with an implant recapture mechanism fluidly connected to the retraction fluid flow circuit.

10. The system of any of claims 8-9, wherein the implant deployment mechanism comprises an elongate flexible catheter attached to the first portion of the manifold, wherein the catheter comprises an internal lumen in fluid communication with a distal outlet of the manifold, an implant advancement piston mounted to the flexible catheter, and a delivery capsule a capsule configured to house the implant, wherein delivery capsule comprises an internal containment element that engages the implant advancement piston, and wherein a reversible flow of the fluid from the lumen of the catheter into a distal chamber of the delivery capsule urges a distal tip of the capsule toward the distal end of the apparatus such that the delivery capsule moves slidably over the implant advancement piston.

11. The system of any of claims 8-10, wherein the implant recapture mechanism comprises an elongate tubular proximal chamber attached to the second portion of the manifold, wherein the proximal chamber comprises an internal passage in fluid communication with a proximal outlet of the manifold, the proximal chamber comprising an internal containment element with an implant retraction piston therein, an wherein flow of the pressurized fluid into the proximal chamber urges the implant retraction piston toward the proximal end of the proximal chamber.

12. The system of claim 11, wherein a distal end of the retraction piston abuts a linear spring and proximal end of the implant retraction piston abuts a stop mechanism configured to limit travel of the implant retraction piston within the containment element of the proximal chamber and toward the proximal end of the apparatus.

13. The system of any of claims 8-12, wherein a tensile member mechanically tethers the delivery capsule to the implant retraction piston such that the capsule and the implant retraction piston move in the same direction.

14. The system of claim 13, wherein pressurized fluid flow into the implant deployment mechanism moves the delivery capsule toward a distal end of the apparatus and biases the implant retraction piston against the linear spring, and wherein pressurized fluid flow into the implant recapture mechanism urges the implant retraction piston in a proximal direction.

15. The system of claim 14, wherein the distal chamber of the delivery capsule comprises a spring that urges the delivery capsule toward the distal end of the apparatus and away from the implant advancement piston.
